# EUROPÄISCHE PATENTSCHRIFT

(11) **EP 0 349 479 B1**
(45) Veröffentlichungstag und Bekanntmachung des Hinweises auf die Patenterteilung: **08.12.1993**
(21) Anmeldenummer: 89730150.3
(22) Anmeldetag: 23.06.1989
(51) Int. Cl.: A61B 1/00, A61C 1/16

(54) **Endoskop**
Endoscope
Endoscope

(30) Priorität: 25.06.1988 DE 8808299 U
(43) Veröffentlichungstag der Anmeldung: 03.01.1990
(73) Patentinhaber: Effner Biomet GmbH, D-12247 Berlin (DE)
(72) Erfinder: Anapliotis, Emmanuel, D-1000 Berlin 33 (DE)
(74) Vertreter: Christiansen, Henning, Dipl.-Ing.

(56) Entgegenhaltungen:
- EP-A- 0 184 778
- DE-A- 3 716 401
- US-A- 4 721 097
- US-A- 4 741 326

## Beschreibung

Die Erfindung betrifft ein Endoskop der im Oberbegriff des Anspruchs 1 angegebenen Art.

Es sind Endoskope bekannt, welche über einen C-Mount-Anschluß mit einer handelsüblichen Videokamera verbindbar sind und es somit in der dentalen Praxis erlauben, dem zu behandelnden Patienten auf einem Videomonitor den zu behandelnden Zahnbereich zu Zeigen, um auf diese Weise die Beratung des behandelnden Arztes zu unterstützen.

Aus hygienischen Gründen ist es notwendig, daß die Reinigung des Endoskops zwischen zwei aufeinanderfolgenden Behandlungen verschiedener Patienten sichergestellt ist. Eine Sterilisation des Endoskops selbst ist sehr aufwendig und würde zu einer unvertretbaren Kostenerhöhung der Behandlung führen.

Es ist ein Schutzüberzug zur Rein- bzw. Sterilhaltung eines Endoskops aus der EP-A-0 184 778 bekannt, welcher das Endoskop umhüllt und am objektnahen, distalen Ende ein für die Beleuchtungs- und Beobachtungsoptik durchsichtiges Sichtfenster aufweist. Nachteilig bei diesem bekannten Schutzüberzug ist aber, daß er nicht wiederverwendbar ist. Zur Verringerung des Rohstoffverbrauchs und zur Vermeidung von schwer entsorgbarem klinischen Abfall sollte aber zur Entlastung der Umwelt die Verwendung von Einwegartikeln stark eingeschränkt werden.

Der Erfindung liegt die Aufgabe zugrunde, ein mit einem Schutztubus versehenen Endoskop anzugeben, bei dem auf einfache Weise eine Vorbeugung gegen Verschmutzung möglich ist, wobei insbesondere der verwendete Schutztubus einfach zu reinigen, wiederverwendbar und kostengünstig herstellbar ist.

Diese Aufgabe wird mit den im kennzeichnenden Teil des Anspruchs 1 angegebenen Maßnahmen gelöst.

Die Erfindung beruht auf der Erkenntnis, daß ein ein Endoskop vor Verschmutzung schützender Schutztubus nach entsprechender Reinigung wiederverwendbar ist und sich trotzdem weiterhin einfach und kostengünstig herstellen läßt, wenn am objektseitigen Ende eine ein Planglaselement aufweisende Halterung in eine Öffnung der Wandung des Schutztubus einfügbar ist, so daß die optische Qualität der Bildbetrachtung gesichert ist. Das objektseitige Ende, an dem das Beleuchtungslicht austritt, ist dabei entweder für Vorausblickoptik an der Stirnfläche des schaftförmigen Schutztubus oder für Winkeloptik an der zylindrischen Wandung des Schutztubus vorgesehen.

Die einfache Form des Schutztubus mit einfügbarer Halterung ermöglicht sowohl dessen einfache Verwendung zum Schutz des Endoskops vor Verschmutzung als auch dessen unproblematische Reinigung, da die Halterung auch bei Bedarf vom Schutztubus entfernbar ist und gegebenenfalls mit einer neuen ein Planglaselement aufweisenden Halterung versehen werden kann.

Bei einer bevorzugten Ausführungsform der Erfindung bildet beim Schutztubus die Fassung, d.h. die Halterung für das optische Element eine Acrylglashülse, welche ihrerseits transparent ist und somit als Durchtrittselement für die Beleuchtung dient, an das geringere optische Anforderungen zu stellen sind.

Ein derartig aufgebauter Schutztubus ist in einfacher Weise herstellbar und somit als kostengünstiger Massenartikel geeignet.

Das Beleuchtungsfeld verbessert sich noch, wenn die Hülse aus Acryl in ihrem stirnseitigen Bereich an der Lichtaustrittsseite kegelstumpfförmig oder linsenförmig in der Weise geformt ist, daß der weiter außen befindliche Bereich, bezogen auf die Lichtaustrittsfläche, zurücktritt.

Eine lichtundurchlässige Innenauskleidung der Acryl-Hülse verhindert Reflexionen und trennt das der Beleuchtung dienende Licht von dem vom Objekt aufgenommenen.

Endoskope mit Winkeloptik sind gemäß einer weiteren bevorzugten Ausführungsform mit einem Schutztubus ausgestattet, bei dem die Halterung als Fassung für ein Planglas in der zylindrischen Wandung des objektzugewandten Tubusschaftes eingefügt ist. Die Halterung ist dabei so geformt, daß sie in einer entsprechenden als Bohrung ausgebildeten Aussparung der Wandung des Schutztubus fest eindrückbar ist. Dazu sind einerseits Auflagewulste vorgesehen, die in Sackbohrungen an der Außenseite der Wandung des Schutztubus eingreifen und andererseits Verdickungen, die in das Innere des Schutztubus hineinragen. Die Halterung besteht vorzugsweise aus einem schwach elastischen Kunststoff- oder Gummimaterial. Der Schutztubus ist für diesen Anwendungsfall stirnseitig verschlossen.

Entsprechend einer vorteilhaften Weiterbildung der Erfindung ist neben der, mit einem Planglas versehenen Halterung für den Durchtritte des optischen Strahlenganges eine zweite derartige Halterung für den Durchtritt des Beleuchtungsstrahlenganges vorgesehen. Die beiden Gläser sind bevorzugt in axialer Richtung hintereinander benachbart angeordnet.

Der Schutztubus ist bevorzugt an dem dem Lichtaustrittsende gegenüberliegenden Ende erweitert, d.h. geht nach einem kegelförmigen Ansatz in einen Bereich größeren zylindrischen Außendurchmessers über, der insbesondere Aussparungen aufweist, welche eine Bedienung des Rändelringes der Endoskop-Optik zur Scharfeinstellung ermöglichen. Desweiteren ist bevorzugt eine Aussparung vorgesehen, welche zum dem Lichtaustrittsende entgegengesetzten Ende hin in eine schlitzförmige Öffnung übergeht, die den Durchtritt des Glasfaserabzweigs zum Anschluß der Lichtquelle zuläßt. Da dieser Anschluß in der Regel als zylindrischer Stutzen ausgebildet ist, kann er als Schnappelement zur Halterung des Schutztubus dienen. Alternativ oder zusammenwirkend mit dieser Schnappvorrichtung ist bevorzugt am dem Lichtaustrittsende gegenüberliegenden Ende eine Hinterschneidung nahe der zylindrischen Öffnung vorgesehen, welche in eine umlaufende Rille oder eine entsprechende Hinterschneidung der Endoskop-Optik eingreift und somit ebenfalls ein Einschnappen zur Halterung des Schutztubus ermöglicht.

Zwei vorteilhafte Ausführungsbeispiele der Erfindung sind in den Figuren dargestellt und werden nachfolgend näher erläutert. Es zeigen:
Figur 1 ein Vorausblickendoskop zur Anwendung mit einem Schutztubus,
Figur 2 einen Schutztubus für ein Endoskop nach Fig. 1,
Figur 3 das vordere Ende des Endoskops gemäß Fig. 1 neben dem entsprechenden Ende des Schutztubus gemäß Fig. 2 in einer Schnittdarstellung,
Figur 4 das vordere Ende eines Schutztubus für ein Endoskop mit abgewinkelter Blickrichtung in einer perspektivischen Darstellung bei schräger Draufsicht sowie
Figur 5 einen Schnitt gemäß der Richtung V - V gemäß Figur 5.

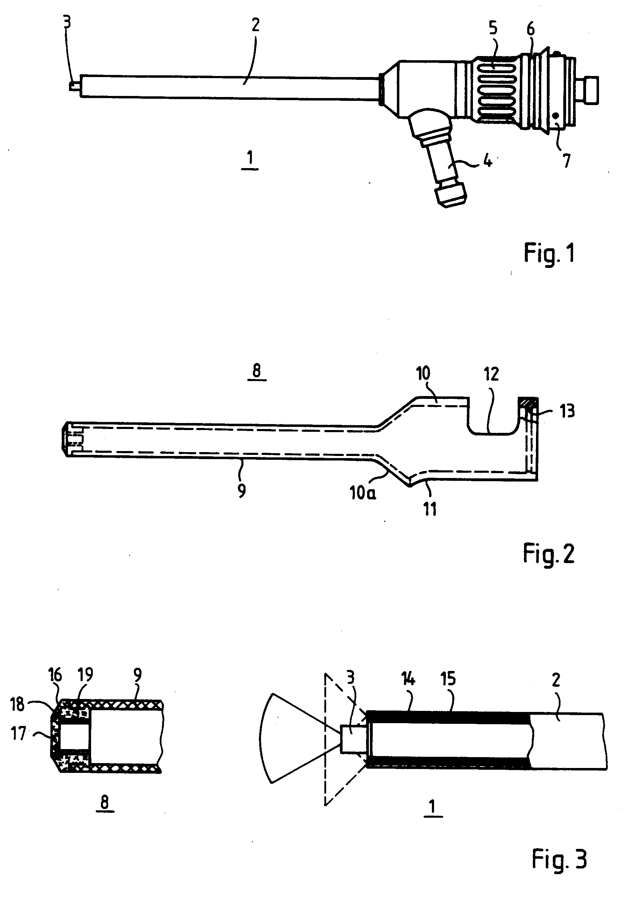

Bei dem in Fig. 1 dargestellten Endoskop 1 für dentale Anwendung ist ein Optik-Hüllrohr 2 vorgesehen, welches in seinem Inneren eine kreisrunde Glasfaseranordnung zur Beleuchtung enthält. An seinem vorderen (Lichtaustritts-)Ende ist ein Objektiv 3 vorgesehen, welches aus dem Lichtaustrittsende des Optik-Hüllrohrs 2 herausragt. Das gegenüberliegende Ende des Endoskops weist einen Anschluß 4 für die Lichtquelle auf, welche seitlich herausragt, sowie einen Rändelring 5 zur Scharfeinstellung der Optik. An den Rändelring schließt ein feststehender, eine Hinterschneidung oder umlaufende Einrillung 6 ausweisender Bereich an, an den ein C-Mount-Anschluß 7 zum Verbinden mit einer Videokamera anschließt.

Der in Fig. 2 dargestellte Schutztubus 8 ist in seinem Innendurchmesser des hohlzylindrischen Bereichs 9 an den Außendurchmesser des Objektiv-Hüllrohrs 2 des Endoskops 1 angepaßt. Der Schutztubus 8 besteht aus PVC und bildet ein Spritzteil oder ist aus einem rohrförmigen Halbzeug durch Nachbearbeitung hergestellt. Ein sich an den an das Objektiv-Hüllrohr 2 angepaßten distaler Bereich 9 anschließender proximaler Bereich 10 weist einen größeren Querschnitt auf. Der proximale Bereich 10 ist aber ebenfalls zylindrisch ausgebildet und zu dem distalen Bereich 9 koaxial ausgerichtet. Der proximale Bereich 10 ist mit dem distalen Bereich 9 über eine kegelstumpfförmige Zone 10a verbunden.

Der proximale Bereich 10 weist eine Ausnehmung 11 auf, welche dem Querschnitt des Stutzens 4 für den Anschluß der Beleuchtungsquelle angepaßt ist. Die Ausnehmung 11 geht in einer Breite, die gegenüber dem Durchmesser des Stutzens 4 im Bereich des Schutztubus 8 angepaßt ist, in einen schlitzförmigen Bereich verringerter Breite über, der sich bis zum der Lichtaustrittsseite gegenüberliegenden Ende des Schutztubus 8 erstreckt. Desweiteren ist eine Ausnehmung 12 vorgesehen, welche bei auf das Endoskop 1 aufgestecktem Schutztubus 8 dem Rändelring 5 benachbart ist, so daß durch diese Öffnung hindurch der Rändelring 5 und damit die Bildschärfe einstellbar ist.

In einem geschnitten dargestellten Bereich nahe der Ausnehmung 12 ist eine innenseitig umlaufende Wulst 13 vorgesehen, welche in die Rille 6 eingreift und somit eine Schnapphalterung für den Schutztubus 8 bildet. Dieser läßt sich leicht vom Endoskop 1 lösen, wenn er mit der Hand umfaßt wird, wobei der Daumen gegen das Anschlußelement 4 drückt und das Endoskop 1 leicht zurückschiebt.

In Fig. 3 sind die vorderen Bereiche vom Endoskop 1 und des Schutztubus 8 vergrößert und im Schnitt dargestellt.

Im geschnittenen Bereich des Objektiv-Hüllrohrs 2 ist die kreisringförmig angeordnete Glasfaseranordnung 14 erkennbar, welche der Lichtführung dient. An der Innenseite des Glasfaserbereichs 14 ist eine lichtundurchlässige Hülse 15 zur optischen Entkopplung vorgesehen. Das aus dem Objektiv-Hüllrohr 2 vorstehende Objektiv 3 ist so angeordnet, daß es beim Aufschieben des Schutztubus 8 in einen dort vorgesehenen Reflexionsschutzring 19 gelangt, dessen Innendurchmesser an den Außendurchmesser des Objektivs 3 angepaßt ist. Dieser Reflexionsschutzring 19 befindet sich im Inneren einer als Acrylhülse 16 ausgebildeten Halterung, welche den Durchtritt des zur Beleuchtung des Objekts dienenden Lichtes von der Glasfaseranordnung 14 her zuläßt. Die stirnseitige Planfläche der Glasfaseranordnung 14 und die innere Stirnfläche der Acrylhülse 16 liegen bei auf das Endoskop 1 aufgestecktem Schutztubus 8 plan aufeinander und ermöglichen damit einen nahezu verlustfreien Lichtdurchtritt und damit eine optimale Objektausleuchtung. Die Acrylhülse 16 weist eine kegelförmig ringförmige Stirnfläche auf, welche eine Brechung des zur Beleuchtung des Objektes aus dieser als Acrylhülse ausgebildeten Halterung austretenden Lichtes in der Weise ermöglicht, daß eine gleichmäßige Verteilung des Beleuchtungslichtes auf den zu betrachtenden Bereich ermöglicht ist.

Am Lichtaustrittsende der Acrylhülse 16 vor dem Reflexionsschutzring 19 ist eine zylindrische Ausnehmung 18 vorgesehen, welche ihren Innendurchmesser zum Lichtaustrittsende hin verringert, also bevorzugt kegelstumpfförmig ausgebildet ist.

Eine Stufung oder dergleichen ist aber entsprechend geeignet, eine Klemmhalterung für ein rundes Planglas 17 zu bilden, das die unverfälschte Betrachtung des Objektes mittels des Objektivs 3 ermöglicht. Dieses Planglas 17 bildet einen Teil der Schutzvorrichtung, wobei im Betrachtungsbereich die Güte der optischen Eigenschaften ein Maximum sein muß, während im Austrittsbereich der Beleuchtung geringere Anforderungen zu stellen sind. Um störenden Lichtaustritt von der Glasfaseranordnung 14 zu verhindern ist bei diesem Ausführungsbeispiele der Schutztubus 8 undurchsichtig. Die dargestellte Halterung für das Planglas 17 in Form der Acrylhülse 16 bildet somit sowohl ein einfaches Halterungselement für das Planglas 17 als auch gleichzeitig ein Lichtaustrittselement im Übergang zum undurchlässigen Bereich des Schutztubus 8. Die damit erreichte konstruktive Optimierung ermöglicht eine Herstellung aus wenigen kostengünstigen Teilen bei einfacher Montagehandhabung. Die Acrylhülse 16 ist bevorzugt in den angrenzenden Bereich 9 des Schutztubus 8 eingepreßt, wobei an den aneinandergrenzenden Flächen eingelassene Rillen oder dergleichen die gegenseitige Verbindung verbessern und gleichzeitig die erzielte Dichtigkeit erhöhen können.

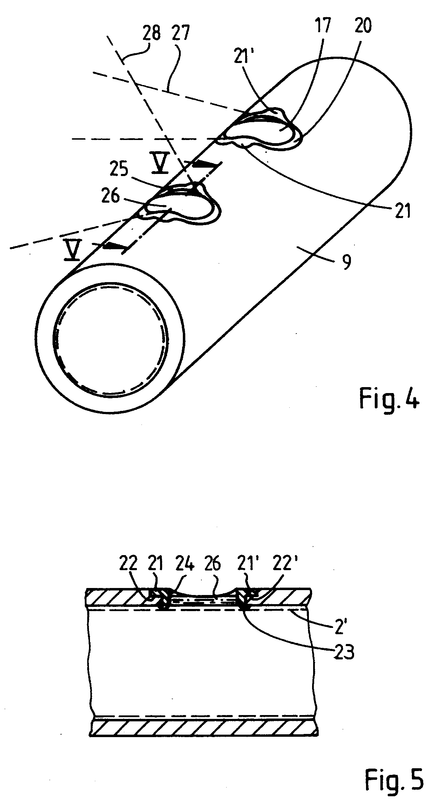

Figur 4 und Figur 5 zeigen ein Ausführungsbeispiel mit einem Endoskop abgewinkelter Blickrichtung in einer perspektivischen Ansicht und im Querschnitt. Bei einem derartigen Endoskop sind Betrachtungsoptik und Beleuchtungsaustrittsöffnung in axialer Richtung hintereinander angeordnet. Entsprechend der Abwinklung des optischen Strahlenganges ist ein Planglas 17 in der Wandung im distalen Bereich 9 des Schutztubus 8 eingesetzt. Als Fassung für das Planglas 17 dient eine Halterung 20, die sich aufgrund materialbedingter Elastizität in die Wandung des distalen Bereichs 9 des Schutztubus 8 sitzfest eindrücken läßt.

Die Halterung 20 ist mit zwei sich an einander gegenüberliegenden Bereichen ohrenförmig erweiterenden Auflagewülsten 21 und 21' versehen, welche in entsprechende Sackbohrungen 22 und 22' an der Außenseite des distalen Bereichs 9 des Schutztubus 8 eingreifen und somit ein Hineinrutschen der Halterung 20 in der distalen Bereich 9 verhindern. Die als Sperrelemente dienenden ohrenförmigen Erweiterungen der Auflagewülste erstrecken sich deshalb in axialer Richtung des Schutztubus 8, da hier die Außenoberfläche des zylindrischen Schutztubus 8 in radialer Richtung den größten Abstand von der Außenfläche des eingesetzten Planglases 17 aufweist und somit eine den Auflagewulst aufnehmende Ausnehmung die größte Tiefe aufweisen kann.

Damit auch ein Herausrutschen der Halterung 20 in Richtung nach außen verhindert wird, ist diese mit einer ringartigen Hinterschneidung 23 in Form einer umlaufenden Dichtlippe versehen, deren eingeschlossene Fläche der Krümmung der Innenfläche des distalen Bereich 9 des Schutztubus 8 angepaßt ist.

Das Planglas 17 ist in einer konischen Ausnehmung 24 der Halterung 20 bis zu einem Anschlag eingepreßt. Eine zweite Halterung 25 trägt ein weiteres Planglas 26, das in seiner Form und Größe der Lichtaustrittsfläche der gebündelten Lichtleitfasern entspricht. Das Planglas 26 ist entsprechend befestigt. Um eine gute Überlappung des Beleuchtungsstrahlenkegels 27 und des Beobachtungsstrahlenkegels 28 zu erreichen, sind die beiden Halterungen 20 und 25 unmittelbar benachbart und axial hintereinander in die Wandung im distalen Bereich 9 des Schutztubus 8 eingefügt.

Die Dichtlippen der Hinterschneidungen 23 liegen jeweils am Schaft des Endoskops an und verhindern einen Lichtübertritt zwischen Betrachtungs- und Beleuchtungsstrahlengang. Dies ist für die Halterung 20 insbesondere in der Schnittdarstellung gemäß Figur 5 mit gestrichelt dargestelltem Optik-Hüllrohr 2' erkennbar.

Die Erfindung beschränkt sich in ihrer Ausführung nicht auf das vorstehend angegebene bevorzugte Ausführungsbeispiel. Vielmehr ist eine Anzahl von Varianten denkbar, welche von der dargestellten Lösung auch bei grundsätzlich anders gearteten Ausführungen Gebrauch machen.

## Patentansprüche

1. Endoskop (1) mit einem beleuchtungs- und optiktragenden (2 und 3) zylinderförmigen distalen Bereich und einem Anschlüsse (4 und 7) für die Beleuchtungsquelle bzw. Einblickoptik aufweisenden proximalen Bereich, und einem Schutztubus (8), der eine Wandung aus elastischem Kunststoff aufweist, die die distalen und proximalen Bereiche des Endoskops (1) umschließt und an dem den proximalen Bereich des Endoskops (1) abgewandten Ende verschlossen ist und die mindestens eine mit einem Fenster zur Beleuchtung und/oder Beobachtung eines Objekts verschlossene Öffnung aufweist,
**dadurch gekennzeichnet,**
daß das Fenster des Schutztubus (8) durch ein in einer Halterung (16, 20, 25) befestigtes Planglas (17, 26) gebildet ist, die unter Ausnutzung der Elastizität der Wandung sitzfest und dichtend auswechselbar in die Öffnung des Schutztubus (8) eingefügt ist.

2. Endoskop (1) nach Anspruch 1, **dadurch gekennzeichnet,** daß das Planglas (17, 26) kreisrund ausgebildet ist und daß die Halterung (16, 20, 25) zur Befestigung des Planglases (17, 26) eine kegelstumpfförmige Ausnehmung (18, 24) in ihrer objektnahen Stirnfläche aufweist, wobei der Innendurchmesser der Ausnehmung (18) sich zur objektnahen Stirnfläche hin verringert und das Planglas (17, 26) vom Bereich mit verringertem Innendurchmesser hintergriffen wird.

3. Endoskop (1) nach einem der vorangehenden Ansprüche, **dadurch gekennzeichnet,** daß die Halterung (16) als ringförmige Acrylhülse ausgebildet ist, deren objektnahen Stirnfläche zum Inneren des Schutztubus (8) hin lichtdurchlässig ist.

4. Endoskop (1) nach Anspruch 3, **dadurch gekennzeichnet,** daß die objektnahe Stirnfläche der ringförmigen Acrylhülse (16) kegelstumpfförmig ausgebildet ist.

5. Endoskop (1) nach Anspruch 4, **dadurch gekennzeichnet,** daß die Innenfläche der ringförmigen Acrylhülse (16) hinter der Ausnehmung (18) für das Planglas (17) zylindrisch und lichtundurchlässig ausgebildet ist.

6. Endoskop (1) nach Anspruch 5, **dadurch gekennzeichnet,** daß die Innenfläche der ringförmigen Acrylhülse (16) mit einem metallenen Reflexionsschutzring (19) versehen ist.

7. Endoskop (1) nach einem der Ansprüche 5 bis 6, **dadurch gekennzeichnet,** daß der Innendurchmesser der Innenfläche der ringförmigen Acrylhülse (16) bzw. der des Reflexionsschutzrings (19) dem Außendurchmesser des Objektivs (3) des Endoskops (1) im distalen Bereich angepaßt ist.

8. Endoskop (1) nach einem der vorangehenden Ansprüche, **dadurch gekennzeichnet,** daß die Wandung des Schutztubus (8) an dem den proximalen Bereich des Endoskops (1) abgewandten Ende eine Öffnung aufweist, in die die als ringförmige Acrylhülse ausgebildete Halterung (16) einsetzbar ist.

9. Endoskop (1) nach einem der Ansprüche 1 bis 2 , **dadurch gekennzeichnet,** daß die Halterung (20, 25) aus einem elastischen Material, insbesondere Kunststoff oder Gummi, besteht.

10. Endoskop (1) nach Anspruch 9, **dadurch gekennzeichnet,** daß die Halterung (20, 25) mindestens einen Auflagewulst (21, 21') aufweist, der in eine in die Außenfläche der Wandung des Schutztubus (8) eingelassene Sackbohrung (22, 22') eingreift, die insbesondere in Längsrichtung der Wandung des Schutztubus (8) größere Abmessungen aufweist als in Querrichtung.

11. Endoskop (1) nach einem der Ansprüche 9 bis 10, **dadurch gekennzeichnet,** daß die Halterung (20) einen in das Innere des Schutztubus (8) hineinragenden, insbesondere lippenartig als optische Dichtung ausgestalteten, Bereich (23) aufweist, dessen Außendurchmesser den Durchmesser der Öffnung zur Aufnahme der Halterung (20) übertrifft.

12. Endoskop (1) nach einem der Ansprüche 9 bis 11, **dadurch gekennzeichnet,** daß die Wandung des Schutztubus (8) mit zwei in axialer Richtung benachbart angeordneten Öffnungen versehen ist, in die jeweils eine Halterung (20 und 25) einsetzbar ist.

13. Endoskop (1) nach einem der vorangehenden Ansprüche, **dadurch gekennzeichnet,** daß der den proximalen Bereich des Endoskops (1) umschließenden Bereich (10) des Schutztubus (8) im Vergleich zum den distalen Bereich des Endoskops (1) umschließenden Bereich (9) des Schutztubus (8) erweitert ausgebildet ist.

14. Endoskop (1) nach Anspruch 13, **dadurch gekennzeichnet,** daß der den proximalen Bereich des Endoskops (1) umschließenden Bereich (10) des Schutztubus (8) mit mindestens einer Ausnehmung (11, 12) versehen ist.

15. Endoskop (1) nach Anspruch 14, **dadurch gekennzeichnet,** daß zwei Ausnehmungen (11, 12) einander diagonal gegenüberliegend angeordnet sind.

16. Endoskop (1) nach einem der Ansprüche 14 bis 15, **dadurch gekennzeichnet,** daß eine vom proximalen Ende des Schutztubus (8) ausgehende schlitzförmige Aussparung (11) einen quer zum Schlitz erweiterten Bereich aufweist.

17. Endoskop (1) nach einem der Ansprüche 13 bis 16, **dadurch gekennzeichnet,** daß das proximale Ende des Schutztubus (8) einen eine Hinterschneidung aufweisenden ringförmig umlaufenden erhabenen Bereich (13) aufweist.

## Claims

1. Endoscope (1) having a cylindrical distal region which carries illumination and optical means (2 and 3) and a proximal region which has connections (4 and 7) for the light source or viewing optic, and a protective tube (8) which has a wall of resilient plastics material which encloses the distal and proximal regions of the endoscope (1) and is closed off at the end remote from the proximal region of the endoscope (1) and which has at least one opening closed off by means of a window for illuminating and/or observing an object,
characterised in that the window of the protective tube (8) is formed by a flat piece of glass (17,26) fixed in a holder (16,20,25), the holder being replaceably inserted in tightly sealed manner in the opening of the protective tube (8) using the resilience of the wall.

2. Endoscope (1) according to claim 1, characterised in that the flat glass (17,26) is of circular construction and in that the holder (16,20,25) for securing the flat glass (17,26) has a frustoconical recess (18,24) in its end face closest to the object, the inner diameter of the recess (18) decreasing towards the end face closest to the object whilst the flat glass (17,26) is gripped by the region of reduced internal diameter.

3. Endoscope (1) according to one of the preceding claims, characterised in that the holder (16) is constructed as an annular acrylic sleeve, the end face of which closest to the object is translucent towards the interior of the protective tube (8).

4. Endoscope (1) according to claim 3, characterised in that the end face of the annular acrylic sleeve (16) closest to the object is of frustoconical configuration.

5. Endoscope (1) according to claim 4, characterised in that the inner surface of the annular acrylic sleeve (16) behind the recess (18) for the flat glass (17) is cylindrical and translucent.

6. Endoscope (1) according to claim 5, characterised in that the inner surface of the annular acrylic sleeve (16) is provided with a metal reflective protecting ring (19).

7. Endoscope (1) according to one of claims 5 to 6, characterised in that the inner diameter of the inner surface of the annular acrylic sleeve (16) or that of the reflective protecting ring (19) is adapted to fit the external diameter of the objective (3) of the endoscope (1) in the distal region.

8. Endoscope (1) according to one of the preceding claims, characterised in that the wall of the protecting tube (8) at the end remote from the proximal region of the endoscope (1) has an opening into which the holder (16) constructed as an annular acrylic sleeve can be inserted.

9. Endoscope (1) according to one of claims 1 and 2, characterised in that the holder (20,25) consists of a resilient material, particularly plastics or rubber.

10. Endoscope (1) according to claim 9, characterised in that the holder (20,25) has at least one abutment bead (21,21') which engages in a blind bore (22,22') provided in the outer surface of the wall of the protective tube (8), this bore having, in particular, larger dimensions in the longitudinal direction of the wall of the protective tube (8) than in the transverse direction.

11. Endoscope (1) according to one of claims 9 to 10, characterised in that the holder (20) has a region (23) projecting into the interior of the protective tube (8) and constructed as a lip-shaped optical seal, in particular, the external diameter thereof exceeding the diameter of the opening for receiving the holder (20).

12. Endoscope (1) according to one of claims 9 to 11, characterised in that the wall of the protective tube (8) is provided with two openings arranged side by side in the axial direction, each adapted to receive a holder (20 or 25) insertable therein.

13. Endoscope (1) according to one of the preceding claims, characterised in that the region (10) of the protective tube (8) enclosing the proximal region of the endoscope (1) is wider than the region (9) of the protective tube (8) enclosing the distal region of the endoscope (1).

14. Endoscope (1) according to claim 13, characterised in that the region (10) of the protective tube (8) enclosing the proximal region of the endoscope (1) is provided with at least one recess (11,12).

15. Endoscope (1) according to claim 14, characterised in that two recesses (11,12) are arranged diagonally opposite one another.

16. Endoscope (1) according to one of claims 14 to 15, characterised in that the slot-shaped recess (11) starting from the proximal end of the protective tube (8) has a widened portion transversely of the slot.

17. Endoscope (1) according to one of claims 13 to 16, characterised in that the proximal end of the protective tube (8) has an encircling elevated region which has an undercast (13).

## Revendications

1. Endoscope (1) comportant une zone distale de forme cylindrique destinée à supporter l'éclairage et l'optique (2 et 3) et une zone proximale présentant des raccords (4 et 7) pour la source d'éclairage et le système optique d'observation, et un tube de protection (8) qui comporte une paroi en matière plastique élastique qui entoure les zones distale et proximale de l'endoscope (1) et est fermée à l'extrémité opposée à la zone proximale de l'endoscope (1) et qui présente au moins une ouverture fermée par une fenêtre pour éclairer et/ou observer un objet,
caractérisé en ce que
la fenêtre du tube de protection (8) est formée par un verre à face plane (17, 26) fixé dans un support (16, 20, 25), ce support étant interchangeable et introduit de manière ferme et étanche dans l'ouverture du tube de protection (8) en exploitant l'élasticité de la paroi.

2. Endoscope (1) selon la revendication 1, caractérisé en ce que le verre à face plane (17, 26) est circulaire et que le support (16, 20, 25) pour fixer le verre à face plane (17, 26) présente un évidement tronconique (18, 24) dans sa face d'extrémité proche de l'objet, le diamètre intérieur de l'évidement (18) diminuant en direction de la surface extérieure proche de l'objet et le verre à face plane (17, 26) étant saisi par l'arrière par la zone à diamètre intérieur diminué.

3. Endoscope (1) selon l'une des revendications précédentes, caractérisé en ce que le support (16) est réalisé comme un manchon en acryl annulaire dont la face proche de l'objet est transparente vers l'intérieur du tube de protection (8).

4. Endoscope (1) selon la revendication 3, caractérisé en ce que la face proche de l'objet du manchon en acryl annulaire (16) est tronconique.

5. Endoscope (1) selon la revendication 4, caractérisé en ce que la face intérieure du manchon en acryl annulaire (16) est cylindrique et opaque derrière l'évidement (18) pour le verre à face plane (17).

6. Endoscope (1) selon la revendication 5, caractérisé en ce que la face intérieure du manchon en acryl annulaire (16) est munie d'un anneau de protection de réflexion (19) en métal.

7. Endoscope(1) selon l'une des revendications 5 à 6, caractérisé en ce que le diamètre intérieur de la face intérieure du manchon en acryl annulaire (16) ou de l'anneau de protection de réflexion (19) est adapté au diamètre extérieur de l'objectif (3) de l'endoscope (1) dans la zone distale.

8. Endoscope (1) selon l'une des caractéristiques précédentes, caractérisé en ce que la paroi du tube de protection (8) présente à l'extrémité opposée à la zone proximale de l'endoscope (1) une ouverture dans laquelle peut être introduit le support en forme de manchon en acryl annulaire (16).

9. Endoscope (1) selon l'une des revendications 1 à 2, caractérisé en ce que le support (20, 25) est en un matériau élastique, en particulier en matière plastique ou en caoutchouc.

10. Endoscope (1) selon la revendication 9, caractérisé en ce que le support (20, 25) comporte au moins un bourrelet d'appui (21, 21') qui est engagé dans un alésage borgne (22, 22') agencé dans la face extérieure de la paroi du tube de protection (8), celui-ci présentant en particulier dans la direction longitudinale de la paroi du tube de protection (8) des dimensions plus grandes que dans la direction transversale.

11. Endoscope (1) selon l'une des revendications 9 à 10, caractérisé en ce que le support (20) comporte une zone (23) faisant saillie à l'intérieur du tube de protection (8), en particulier en forme de lèvre, à des fins d'étanchéité optique, et dont le diamètre extérieur dépasse le diamètre de l'ouverture dans laquelle est logé le support (20).

12. Endoscope (1) selon l'une des revendications 9 à 11, caractérisé en ce que la paroi du tube de protection (8) présente deux ouvertures adjacentes en direction axiale dans lesquelles peuvent être respectivement introduits les supports (20 et 25).

13. Endoscope (1) selon l'une des revendications précédentes, caractérisé en ce que la zone (10) du tube de protection (8) entourant la zone proximale de l'endoscope (1) est réalisée plus large en comparaison avec la zone (9) du tube de protection (8) entourant la zone distale de l'endoscope (1).

14. Endoscope (1) selon la revendication 13, caractérisé en ce que la zone (10) du tube de protection (8) entourant la zone proximale de l'endoscope (1) comporte au moins un évidement (11, 12).

15. Endoscope (1) selon la revendication 14, caractérisé en ce que deux évidements (11, 12) sont disposés en diagonale l'un par rapport à l'autre.

16. Endoscope (1) selon l'une des revendications 14 à 15, caractérisé en ce qu'un évidement (11) en forme de fente partant de l'extrémité proximale du tube de protection (8) présente une zone élargie transversalement à la fente.

17. Endoscope (1) selon l'une des revendications 13 à 16, caractérisé en ce que l'extrémité proximale du tube de protection (8) présente une zone annulaire circonférentielle en relief présentant une contre-dépouille.
